# EUROPEAN PATENT APPLICATION

(11) **EP 4 482 171 A1**
(43) Date of publication of application: **25.12.2024**
(21) Application number: 23180363.6
(22) Date of filing: 20.06.2023
(51) Int. Cl.: H04R 1/24, H04R 3/00, H04R 3/04, H04R 17/00, H04R 29/00, A61M 5/145, F04B 43/04, H04R 1/42, A61M 5/168

(54) **MEMS DEVICE AND METHOD FOR OPERATING A MEMS DEVICE**

(71) Applicant: Infineon Technologies AG, 85579 Neubiberg (DE)
(72) Inventor: BRETTHAUER, Christian, 81675 München (DE); EVERSMANN, Bjoern Oliver, 94501 Aidenbach (DE); HAUBOLD, Marco, 01309 Dresden (DE); HEISS, Heinrich Guenther, 81373 München (DE); BERNACCHIA, Giuseppe, 9500 Villach (DE); WIESBAUER, Andreas, 9210 Poertschach (AT); MAYRHOFER, Dominik, 8010 Graz (DE)
(74) Representative: König, Andreas Rudolf

(57) **Abstract**

A MEMS device comprises a cavity for emitting a fluid and a pump structure configured for generating a pressure of the fluid in the cavity and for providing the fluid under pressure. The MEMS device comprises a sensor unit configured for sensing an electrical parameter that is based on an electrical impedance of the pump structure. The sensor unit provides a sensor signal based on the electrical parameter. The MEMS device comprises a control unit configured for controlling an operation of the pump structure. The control unit is configured for processing the sensor signal for adapting an operation of the MEMS device and/or for failure detection.

## Description

The present disclosure relates to a MEMS device and to a method for operating a MEMS device, in particular a MEMS device for producing pressure in a fluid, e.g., a speaker. The present disclosure in particular relates to a combination of a pumping speaker with an impedance sensor.

Pumping speakers enable a high level of miniaturization and bass performance and accuracy respectively high sound pressure level at low frequencies and thus very low bass corner frequency, e.g., in true wireless speakers, TWS, as membrane size and deflection respectively displacement can be traded versus vents to direct air flow inpumping operation to accumulate high bass pressure over a multitude of pumping cycles with a compact membrane and compact MEMS enabling further in-ear speaker miniaturization.

Such pumping speakers may be fabricated in a micro electro mechanical system, MEMS, technology using, for example, semiconductor technology materials such as silicon, dielectrics, oxides, metals, or the like. A pumping speaker may accumulate pressure and may use a vent to provide the pressure.

The accumulated pressure may be generated, for example, using a membrane structure that may be obtained, for example, using processors and technologies adapted from producing a MEMS microphone and/or MEMS loudspeakers.

There is a need to provide for a precise operation of a MEMs device and for MEMS devices that may be operated precisely. The goal for speakers and audio devices is precise, balanced audio reproduction over a high frequency and (amplitude) dynamic range. In this context, the adaptation of the control to system properties such as resonances, attenuations or losses is mandatory and thus the need to determine these system properties arises.

An object solved by the present disclosure is therefore to provide a MEMS device and a method to operate a MEMS device that allow for a precise operation of the MEMS device.

According to an embodiment, a MEMS device comprises a cavity for emitting a fluid. The MEMS device comprises pump structure configured for generating a pressure of the fluid in the cavity and for providing the fluid under pressure. A sensor unit of the MEMs device is configured for sensing an electrical parameter that is based on an electrical impedance of the pump structure than for providing a sensor signal based on the electrical parameter. The MEMS device comprises a control unit configured for controlling an operation of the pump structure. The control unit is configured for processing the sensor signal and for adapting an operation of the MEMs device and/or for failure detection. Sensing and evaluation of the electrical impedance allows for a simple and robust and nevertheless precise evaluation of the operation of the MEMS device such that the adaption of the operation and/or the failure detection may be implemented precisely.

According to an embodiment, a method for operating a MEMS device comprises generating a pressure of a fluid in a cavity of the MEMS structure using a pump structure for providing the fluid under pressure. The method comprises sensing an electrical parameter that is based on an electrical impedance of the pump structure. In the method, a sensor signal is provided based on the electrical parameter. The method comprises controlling an operation of the pump structure and processes the sensor signal for adapting an operation of the MEMS device and/or for failure detection.

Advantageous embodiments in accordance with the present disclosure are defined in dependent claims.

Advantageous embodiments are described hereinafter whilst referring to the accompanying drawings in which:
- Fig. 1: shows a schematic block diagram of a MEMS device according to an embodiment;
- Fig. 2: shows a schematic side view of a MEMS device according to an embodiment;
- Fig. 3a-b: show schematic side views of a part of MEMS devices according to embodiments that illustrate the concept of measuring the electrical parameter at the volume displacing structure;
- Fig. 4: shows a schematic side view of a MEMS device according to an embodiment, the MEMS device comprising one or multiple conventional membrane-based MEMS speaker tweeter structures;
- Fig. 5: shows a schematic side view of a MEMS device according to an embodiment having a pressure sensor;
- Fig. 6: shows a schematic flow chart of a process according to an embodiment;
- Fig. 7a: shows a schematic block diagram of an implementation example of a combined sensing and driver circuit in connection with an embodiment; and
- Fig. 7b: shows a schematic representation of a plot over a time at the ordinate to illustrate a result of a sense signal such in accordance with embodiments.

Equal or equivalent elements or elements with equal or equivalent functionality are denoted in the following description by equal or equivalent reference numerals even if occurring in different figures.

In the following description, a plurality of details is set forth to provide a more thorough explanation of embodiments of the present disclosure. However, it will be apparent to those skilled in the art that embodiments of the present disclosure may be practiced without these specific details. In other instances, well known structures and devices are shown in block diagram form rather than in detail in order to avoid obscuring embodiments of the present disclosure. In addition, features of the different embodiments described hereinafter may be combined with each other, unless specifically noted otherwise.

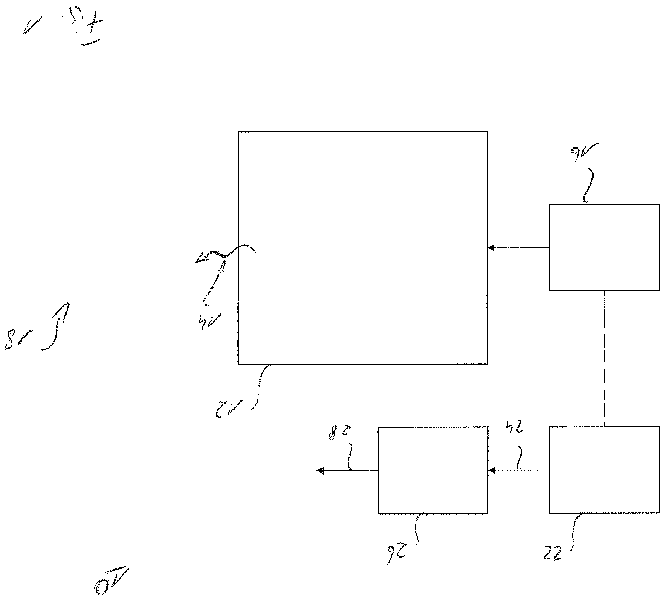

Fig. 1 shows a schematic block diagram of a MEMS device 10 according to an embodiment. The MEMS device 10 comprises a cavity 12 for emitting a fluid 14. The fluid 14 may be a liquid or a gas and may be subject to a pressure generated by a pump structure 16 of the MEMS device that generates the pressure of the fluid 14 in the cavity 12 to provide the fluid 14 under pressure. Although describing at some of the embodiments in connection with a speaker implementation that generates a pressure in a gas, e.g., air, the embodiments are not limited hereto. Other gases, e.g., of a single type or mixtures of gases such as in the air, may be contained in the cavity 12 as well as fluids such as conductive or non-conductive fluids.

The fluid 14 may be provided under pressure to an environment of the MEMS device 10 or to an intermediate volume. For example, such a volume 18 may be an internal volume of the MEMS device 10 or an outer volume such as an in-ear volume respectively ear channel of a listener.

The MEMS device 10 comprises a sensor unit 22 configured for sensing an electrical parameter of the pump structure 16. The electrical parameter may be based on an electrical impedance of the pump structure 16. The sensor unit 22 may be configured for providing a sensor signal 24 based on the electrical parameter. That is, the sensor signal 24 may comprise an analog or digital value that is directly or indirectly proportional to the electrical impedance. Alternatively or in addition, the sensor signal 24 may comprise information, e.g., in an analogous or digital manner, that indicates the electrical impedance or a value derived thereof. An example of a value derived thereof may be a normalized value of the electrical impedance of change of impedance, e.g., being normalized to a scale having a minimum value and a maximum value. Another example of a value derived thereof may be a specific change or a transient behavior or signature of the measured value as e.g. a settling characteristic or the like (during constant or altered operation conditions, which can be part of calibration procedures). Other types of signal processing or processing of measurement values is possible, without any limitation, in the sensor unit 22.

The MEMS device 10 may comprise a control unit 26 configured for controlling an operation of the pump structure 16. The operation of the pump structure 16 may relate to an amplitude of pressure being generated, an amplitude of a movement of a moveable element of the pump structure 16, an operating frequency of at least an element of the pumping structure 16 and/or a timing of a movement or the like. The control unit 26 may be configured for processing the sensor signal 24 for adapting an operation of the MEMS device 10 and/or for failure detection. That is, the sensor signal 24 may comprise information indicating at least a parameter that is linked to the operation of the pump structure. For example, a change in the electrical impedance, a frequency of the change, a pattern with in the sensor signal 24 or other parameters may allow for a comparison and/or for a determination, e.g., using a determination rule, whether the operation of the pump structure 16 is within predefined boundaries. Alternatively or in addition, the control unit 26 may determine an operating frequency of the pump structure 16 and may adjust a timing of an operation of further elements of the MEMS device 10. For example, a valve structure or the like may be used to control an exit or emittance of the fluid 14 out of the cavity 12. Such an operation may be partly arranged or synchronized to an operation of the pump structure 16. Alternatively or in addition, e.g., one or more properties of the sensor signal 24 exceeding predefined values or a result of a determination rule exceeding a predefined threshold may indicate that the MEMS device 10 or a part thereof, e.g., the pump structure 16 or a valve structure or the like, shows a behavior beyond a target operating condition. This may provide for a source of determining an optimum operation condition as well as deviations from optimum operation conditions and/or failures. The control unit 26 may, for example, provide for a signal 28 that indicates a failure and/or alert and/or that indicates an adaptation of operation of at least one element of the MEMS device 10.

According to an embodiment, the control unit 26 comprises an application specific integrated circuit, ASIC. The sensor unit 22 may form a part of the ASIC and or MEMS. An implementation of the control unit 26 and the sensor unit 22 as two dedicated elements or structures in the MEMS device is possible, with argumentation.

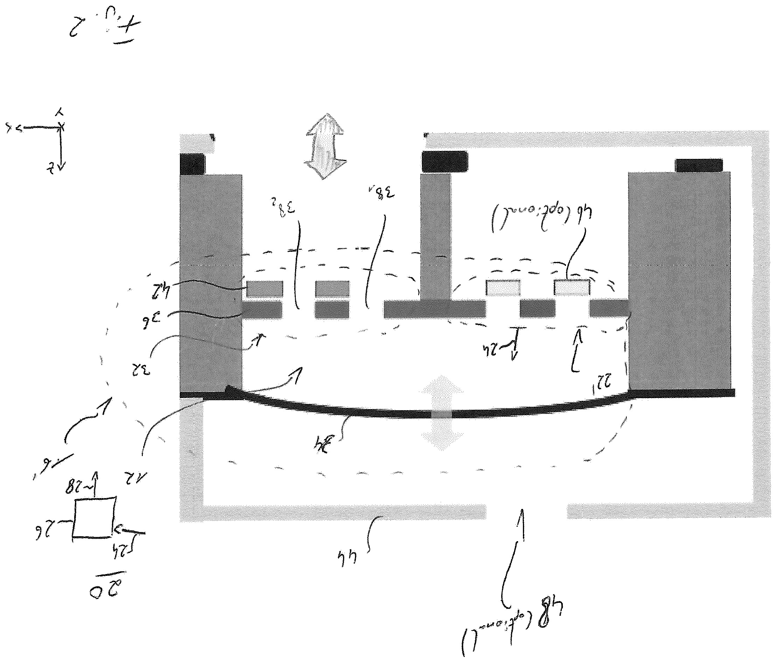

Fig. 2 shows a schematic side view of a MEMS device 20 according to an embodiment. A pump structure 16' comprises a valve structure 32 and a non-perforated or perforated volume displacing membrane or structure 34 configured for generating the pressure in the cavity 12 and for releasing the fluid by switching between a first state of the valve structure 32 in which the valve structure provides a first fluid resistance for the fluid and a second state in which the valve structure provides a second fluidic resistance for the fluid. The first fluidic resistance is higher when compared to the second fluid resistance. That is, in different states different levels of fluid resistance may be provided by the valve structure 32. The transition between the states can be discrete in nature or (more likely) will be highly continuous. According to one example, the valve structure 32 may be switched frequently between a closed state and an open state. However, the valve structure 32 is not required to switch between discrete states like a completely closed state and a completely open state and may implement other concepts of modulating a fluid resistance. By providing a higher fluidic resistance in the second state, the volume displacing structure 34, e.g., a membrane structure or other type of moveable element that is movable in-plane or out-of-plane of the MEMS device 20, pressure may be accumulated in the cavity 12 and the fluid comprising the pressure may be released through the valve structure 32, at least in a state in which the valve structure 32 shows the lower fluidic resistance for the fluid.

For example, the valve structure 32 may comprise a shutter structure, e.g., a perforated stator structure comprising one or more passages such as wholes through the stator structure 36. One or more holes 38, and/or 38₂ may be at least partially closed or decreased inside by a movable structure 42 that is movable with regard to the stator structure 36.

The movable structure 42 may be movable in-plane, e.g., along an x-direction and/or y-direction or in a rotary or bending movement relative to the stator structure 36 to at least partly close or block openings, slits or holes 38, and/or 38₂ in the first state when compared to the second state. However, such a movement may also be provided, as an alternative or in addition, along an out-of-plane direction of the MEMS device 20, e.g., along a z-direction or in a rotary or bending movement.

The MEMS device 20 may comprise a housing which houses the pump structure 16.

The MEMS device 20 may comprise a sensor unit. For example, the sensor unit may be configured for determining a measurement value from an electrical parameter that is associated with an electrical impedance. For example, the sensor unit may be configured for determining the measurement value based on the knowledge of a functional dependency of the respective electrical impedance and the operation of the MEMS device. By way of on-limiting example, the sensor unit may be configured for determining a resonance frequency of the volume displacing structure 34. Alternatively or in addition, a sensor unit 22' may be configured, for example, to evaluate an electrical impedance between electrodes integrated within a same or a different stator structure 36. For example, sensing mechanisms such as electrodes may be arranged at a pair or a set of structures. For example, at least one first electrode may be arranged at a stator structure such as stator structure 36 and at least one electrode may be arranged at a moveable structure such as the shutter structure 36 and/or 46.

Without limiting the scope of the embodiments, one specific configuration is described in Fig. 2. The shutter structure 42 that is configured to move or deflect along one or more of the group of directions x, y and z and/or a rotary direction ϕ around at least one of the x-axis, the y-axis and the z-axis responsive to an excitation, momentum, spring forces, damping and pressure generated in the cavity 12.. As energy is exchanged in dynamic operation between mechanical energy (e.g. related to shutter momentum, spring forces, pressure forces, viscous effects, ...) and electrical energy e.g. electrical field between capacitor plates such as actuation (or sensing electrodes e.g. of electrostatic or piezo-electric nature) changes with respect to movement characteristics as non-resonant versus less versus non-resonant movement including differences when jolting or blockage occurs may effect strongly the system or electrical characteristics respectively impedance. Such change in the electrical impedance, e.g., related to a capacitance between the shutter structure 46 and the stator structure 36, each carrying an electrode structure at least in the region of the sensor structure 22' may allow to determine and/or monitor the movement characteristics and/or a behavior of the pump structure 16'.

The structure 22' may be an additional, optional vent structure that can increase the efficiency of the pump and ease the explanation of the operation principle of the pump. Also it's operation can be sensed. The housing 44 may comprise one or more optional openings 48 adapted for a pressure compensation in the housing. An air flow 45 may be bi-directional to enable efficient pressure modulation required for sound creation and avoiding the saturation or full depletion of ear channel and at least at audio frequencies sealed reservoir of the device package (the so called back volume).

A frequency of movement of the volume displacing structure 34 may be operated at the same or higher, e.g., higher by a factor of 2, or more, when compared to a frequency or speed of switching between the first state and the second state with the valve structure 32. That is, the pressure may be accumulated in the cavity 12 and the accumulated pressure may be released by use of the valve structure 32.

Measuring the electrical parameter at the valve structure 32, e.g., using the shutter structure 46 interacting with the stator structure 36 is one option of implementing embodiments described herein. It is to be noted that, as an alternative or in addition, the sensor unit 22' may be configured for sensing the electrical parameter between the stator structure 36 and the movable structure 42. For example, another shutter structure and/or valve structure may be optionally used for operation of the MEMS as a pump. Also such an embodiment may use a measurement of an impedance-related value between stator and moveable structure, whether applied to either or both valve structures.

As an alternative, or in addition to measuring the electrical parameter at the valve structure, according to an embodiment, a sensor unit may be configured for measuring the electrical impedance at the volume displacing structure 34. The control unit 26 may be configured for controlling at least one of a timing of a movement of the volume displacing structure 34 with respect to a timing of a movement of the valve structure 32, in particular, the movable structure 42. As an alternative or in addition, the control unit may be configured for controlling a timing of a movement of the valve structure 32, e.g., the movable structure 42, with respect to a timing of a movement of the volume displacing structure 34. Alternatively or in addition, the control unit 26 may be configured for controlling an operation frequency or phase relationship of the pump structure 16, e.g., an operation frequency of the movable structure 34 and/or of the valve structure 32. As an alternative or in addition, whilst not precluding other controls or fault/error-signals, the control unit 26 may be configured for controlling the drive resp. modulated excitation e.g. an operational voltage, current, or charge amplitude, timing, or phase of the MEMS device 20, e.g., to adapt an operation mode or point e.g. an amplitude of movement, timing, or phase relationship, position, or the like.

In Fig. 2 there is shown that not only the membrane structure 34 is moving during operation of the MEMS device 20 but also elements of the valve structure 32, e.g., the shutter structure 42 and/or other elements of the MEMS 20, e.g., the shutter structure 46. Whilst embodiments do not prevent to determine information related to or associated with the operation frequency of the membrane structure 34, embodiments benefit from precise knowledge about the operation of the at least one valve structure. This may allow to adjust an operation of the membrane structure and/or of the vent structure to match a desired operation condition. For example, the valve structure 32 may be continuously operated and it may be desired to operate the valve structure at or close to the resonance frequency. For example, it may be desired to deviate, from the resonance frequency, by a certain possibly device-dependent difference, e.g., at most 10 Hz, at most 20 kHz or at most 40 kHz. As an alternative or in addition to such an absolute value, a relative value such as at most 0.1 %, at most 10 % or at most 50 % of the resonance frequency may be a desired value of avoiding an operation at exactly the resonance frequency whilst still making benefit from the high efficiency of operation. This may allow for even an optimum of a trade-off between harmonic distortion on the one hand and efficient operation on the other hand.

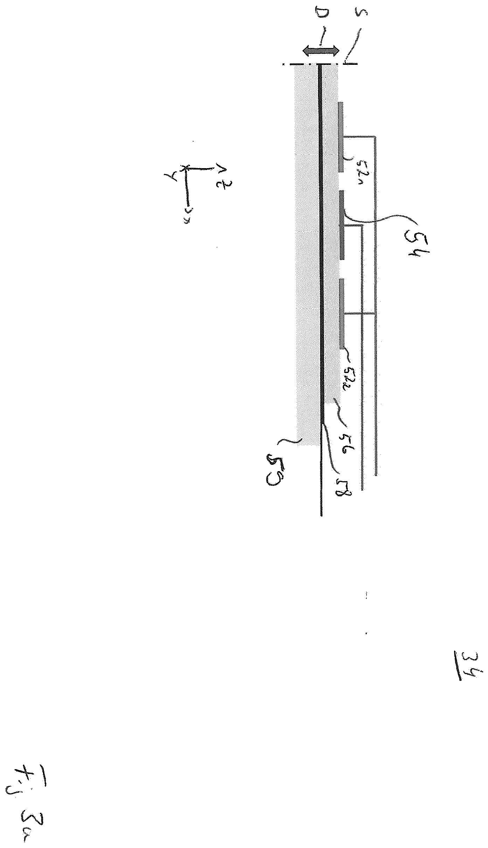

Fig. 3a shows a schematic side view of a part of a MEMS device 20 according to an embodiment that illustrates the concept of measuring the electrical parameter at the volume displacing structure 34. For measuring the electrical parameter, one or more force electrodes 52₁, 52₂, ..., may be attached to the volume displacing structure 34. Optionally, as an alternative or in addition, a sense electrode 54 may be arranged at the volume displacing structure 34. For example, the at least one optional sense electrode 54 and/or the force electrode 52, and/or 52₂ may sandwich an actuating structure 56 together with a ground electrode 58 such that the actuating structure 56 is arranged between the reference electrode 58 on the one hand and other electrodes 52, 54 on the other hand.

Although some embodiments are described to operate a piezoelectric actuator as a volume displacing structure such as structure 34, the volume displacing structure and/or the vent/valve structure can be of electro-static and/or piezo-electric type. Either combination is in accordance with embodiments. For example, in an electro-static implementation a gap instead of the actuating structure 56 may be present between the electrodes (on a second substrate) and may be filled with air, while for piezo-electric actuators the gap is filled with piezo-electric dielectric 56.

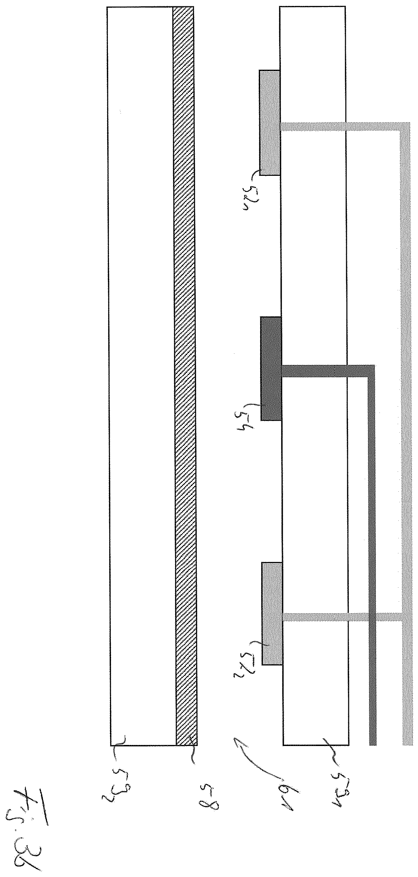

Fig. 3b shows a schematic side view of a part of a MEMS device according to an embodiment that illustrates the concept of measuring the electrical parameter by use of electrodes. For measuring the electrical parameter, the one or more electrodes 52₁, 52₂, and 54, may be attached to a first carrier substrate 59, and oppose the reference electrode 58 spaced by an air gap 61. In other words, the volume displacing actuator (as well as vent) structure may be of electro-static and/or piezo-electric nature. Either combination is covered by embodiments. In the shown electro-static case the gap 56 between the electrodes may be filled with a gas such as air, while for piezo-electric actuators like in Fig. 3a the gap may be filled with the piezo-electric dielectric 56.

In each implementation, the control unit may be configured for evaluating a voltage a charge and/or a current, e.g., at electrodes forming an electrical capacitor structure of or within the pump structure. However, a voltage may also be evaluated at structures different from a capacitor structure. Other measurement values related to the electrical impedance may be measured in addition or as an alternative.

The volume displacing structure 34 may optionally comprise a substrate 59 providing for an additional layer. The actuator structure 56 may comprise, for example, a piezo actuator, an electrostatic actuator, an electrodynamic actuator, a thermal actuator or other structures to provide for a deflection d along a positive and/or negative z-direction. As an alternative or in addition, a movement may also be possible along the x-direction and/or the y-direction as well as in view of a rotational movement and/or a tilting movement. An arrangement of electrodes 52, 54 and/or 58 and/or an arrangement of the actuator structure 56 is possibly symmetric with regard to a symmetry access or symmetry plane S or not. Deflection D may allow for sensing variable capacitances or generated charges, i.e., values based on the electrical impedance or charges based on piezo-electric effect, between the reference electrode 58 on the one hand and the force electrode 52₁/52₂ on the other hand, between the reference electrode 58 and the optional sense electrode 54 on the other hand. For example, when the operation condition allows to use force electrode 52, and/or 52₂ at least during some instances of time for generating information of the sensor unit 22, the one or more sense electrode 54 may be optional or maybe absent. As an alternative, to provide for a higher degree of information, an additional electrode may be beneficial.

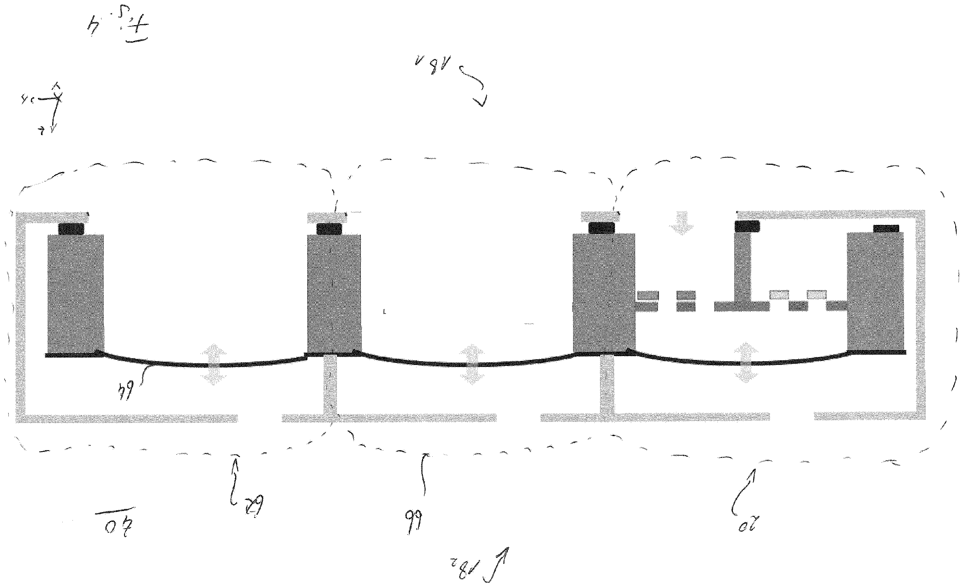

Fig. 4 shows a schematic side view of a MEMS device 40 according to an embodiment. When compared to the MEMS device 20, the MEMS device 40 may additionally comprise one or multiple membrane-based MEMS speaker tweeter structures 62, e.g., based on a conventional membrane. When compared to the MEMS structure 20 that may be configured, for example, to provide for pressure in the fluid, e.g., sound, with a comparatively low frequency to provide for a high level of bass experience, the tweeter structure 62 may operate an associate volume displacing element 64, e.g., a membrane structure, with a comparatively higher frequency when compared to sound emitted by the MEMS device 20. Thereby, to the same or a different volume, sound of a different, e.g., higher, frequency range may be provided, e.g., above 200 Hz, above 500 Hz, or above 1 kHz. Such frequencies may be provided with a sufficient or even high quality by the use of the comparatively small sizes of MEMS membranes. On the other hand, such small membranes may be used along with a valve structure of the MEMS device 20 to provide sound pressures at comparatively low frequency ranges in a bass frequency range of, e.g., at most 2000 Hz, at most 200 Hz or even lower, e.g., 50 Hz.

Optionally, the MEMS device 40 may comprise a pressure sensor structure 66 that is configured for sensing a pressure in an outer volume 18₁ and/or 18₂, e.g., a front volume, a back volume, respectively, to provide for additional input of the sensor unit 22. According to an embodiment, a MEMS device comprises a pressure sensor such as pressure sensor 66, configured for providing a sensor signal that is based on a pressure sensed by the pressure sensor. Evaluating the pressure may allow for evaluating whether a desired amount of pressure is generated and may provide for a basis to adjust the operation of the MEMS device based on the sensed pressure, e.g., to adapt an operation frequency, an amplitude of the pressure or the like.

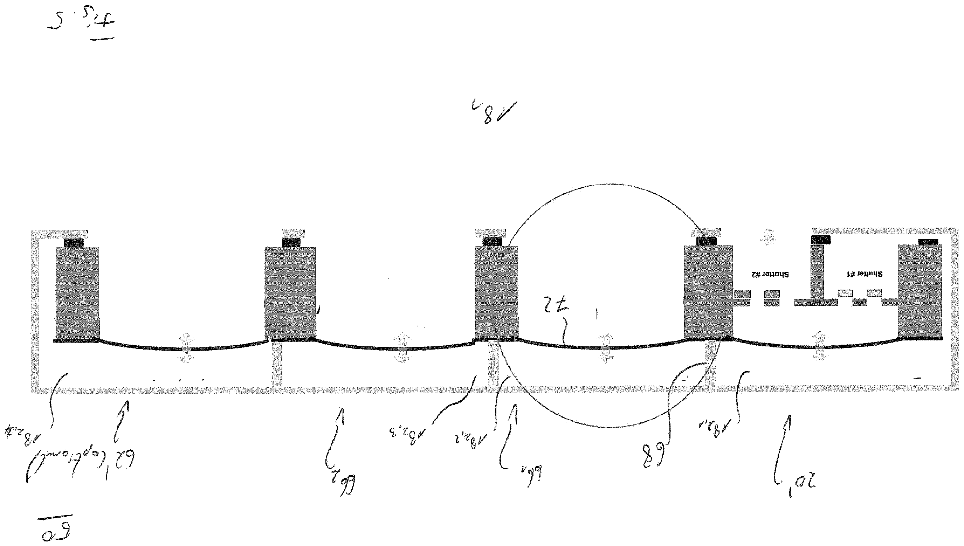

Fig. 5 shows a schematic side view of a MEMS device 20 according to an embodiment. When compared to the MEMS device 40, a tweeter structure 62' has an enclosed back volume 18_{2,4}. Similarly, a pressure sensor 66₂ may comprise an enclosed back volume 18_{2,3} which may allow to focus the pressure sensing on the common front volume 18₁. The sensor unit 66₂ may be configured for sensing a pressure variation in a frequency range beyond the audible range, e.g., in infra sound and/or ultra-sonic sound. For example, a pressure sensor can be integrated into a pumping speaker and/or a combo along with a tweeter, e.g., a respective MEMS.

When compared to the MEMS device 20, a MEMS device 20' forming a part of the MEMS device 50 may comprise a back volume 18_{2,1} that may be connected with a back volume 18_{2,2} through an internal opening or internal fluid passage 68 such that the pressure sensor structure 66, having a deflectable element 72, such as a membrane structure, may operate as a differential pressure sensor in communication with the MEMS device 20'. Thereby, different degrees and/or dimensions of information may be obtained with the sensor unit.

With regard to an operation of the control unit 22 or 22', same may be adapted for measuring the electrical parameter, e.g., once or as a single measurement value or, as an alternative, by measuring a plurality of instances of the electrical parameters. Providing one or more sensor signals related to a plurality of instances of the electrical parameter may allow the control unit to relate the plurality of instances of measurement results of the electrical parameter with each other. For example, when relating the plurality of instances of measurement values, the control unit may be configured to obtain a signature of the measurement values. As a signature, one may understand a signature respectively temporal behavior of the measurement values. That is, for example, the control unit may be configured to determine or derive information that indicates a condition of the pump structure by comparing measurement results related to different time instances or time intervals. For example, the control unit may be configured for evaluating the signature to determine a target operation and for adjusting the operation based on the signature. As a target operation, the control unit may determine a deviation from a target state or may determine a target state to which the MEMS device is to be controlled. One element that may be evaluated, amongst others, is, for example, a switched-capacitor or inductive converter like a charge pump being used to operate the volume displacing element of the pump structure. For example, a number of pulses on the one hand and/or a time required on the other hand that is needed to generate a certain amount of voltage or pressure may indicate the operation condition of the pump structure and tuning to desired target operation. Such a result may indicate a timing to be used, e.g., for controlling the valve structure and/or for adjusting a timing of the pump structure with regard to the above structure. Alternatively or in addition, such information may indicate a target condition of the MEMS device or may be compared against a target condition. For example, deviations in a reference pressure may lead to a higher amount of charge to be used to generate specific pressures and/or specific deflections of the membrane structure.

The signatures determined may be used by the control unit for adjusting the operation.

According to an embodiment, the control unit may be configured for evaluating a number of pulses provided by an electrical source to drive the pump structure. The number of pulses may form a basis for the control unit to determine the signature and the control unit may derive, from the signature, information related to a settling behavior of the pump structure. The pulses may be provided, e.g., by a charge pump or other sources of the MEMS device, cyclically, e.g., with a frequency of at least 40 kHz and at most 400 MHz, at least 100 kHz and at most 1 MHz or at least 400 kHz and at most 600 kHz, e.g., 500 kHz.

Alternatively or in addition, according to embodiments, the frequency may be in one of a set of frequency ranges where the lower and the upper frequency increase, e.g., in a range of at least 40 kHz and at most 12 MHz; of at least 100 kHz and at most 30 MHz; or of at least 400 kHz and at most 100 MHz. The lower limit may be used for a so-called fpumpMEMS and the higher limit may refer to the charge pump operation frequency, i.e., fpumpMEMS < f < fchargepump

However, those values are examples only to illustrate the beneficial operation of the described embodiments. The operation frequency of the electrical source or electrical circuit, e.g., charge pump, may exceed the operation frequency of the acoustical pump very significantly. Moreover it is possible that the charge pump is operated at a constant or a varying frequency during the pumping cycle. Such a variation may be controlled with a control unit such as control unit 22 or 22' between the described frequency values, e.g., between at least 40 kHz and at most 400 MHz, at least 100 kHz and at most 1 MHz or at least 400 kHz and at most 600 kHz or parts of such ranges.

The settling behavior of the sensor e.g. charge pump driver circuit readout may indicate external influences acting on the pump structure or the cavity.

According to an embodiment, the control unit may be configured for determining different signatures for different operating frequencies of the pump structure and for comparing the different signatures to obtain an evaluation result. The control unit may be configured for adapting the operation of the MEMS device and/or for failure detection based on the evaluation result. For example, by operating the pump structure with different operation frequencies, the sensor unit may indicate, with the sensor signal, if the operation is at least in the region of a preferred operating condition. For example, resonances or the like may be evaluated, e.g., when additionally evaluating the deflection of the membrane, vent or actuator structure.

The examples described above illustrate a set of different implementations of the sensor unit that may be combined with one another without limitations. The sensor unit in accordance with embodiments may sense one or more parameters of a group of parameters that includes a voltage, a current and/ or a charge between electrodes of the pump structure, an impedance between electrodes of the pump structure, e.g., a capacitance, and a parameter related to an electrical charge of the pump structure to provide the sensor signal. The parameter related to the electrical charge may comprise, for example, the signature itself or a count of pulses or the like.

As general example a lower impedance and thus higher load requires more current and thus charge transfer to achieve e.g. a certain threshold voltage in a charging process of a reactive resp. capacitive load or another circuit. Thus for a given charge-pump transferring with each charging step some charge, more charging steps can be required, to achieve a certain threshold level in presence of a higher output load and lower impedance. Thus, the number of charge transfers and pulses can be used as indicator of impedance, a relative change thereof and/or a transient impedance behavior and/or signatures.

Thus, the number of pulses can be used as impedance and settling indicator independent, whether the charge-pump directly supplies a transducer load or provides a sub-regulated voltage supplying another circuit, driving the transducer.

Embodiments described herein relate, in general, to a MEMS device that comprise a pump structure to provide pressure to a fluid in a cavity. According to an embodiment, the pump structure may be a part of a loudspeaker structure configured for providing a first sound in the fluid having a first frequency range. The MEMS device, e.g., MEMS device 40 and/or 50, may comprise a membrane structure configured for providing a second sound in a second frequency range higher than the first frequency range and based on a deflection of the membrane structure. The MEMS device may be configured for providing a combination of the first, e.g., low, sound and the second, e.g., high, frequency sound.

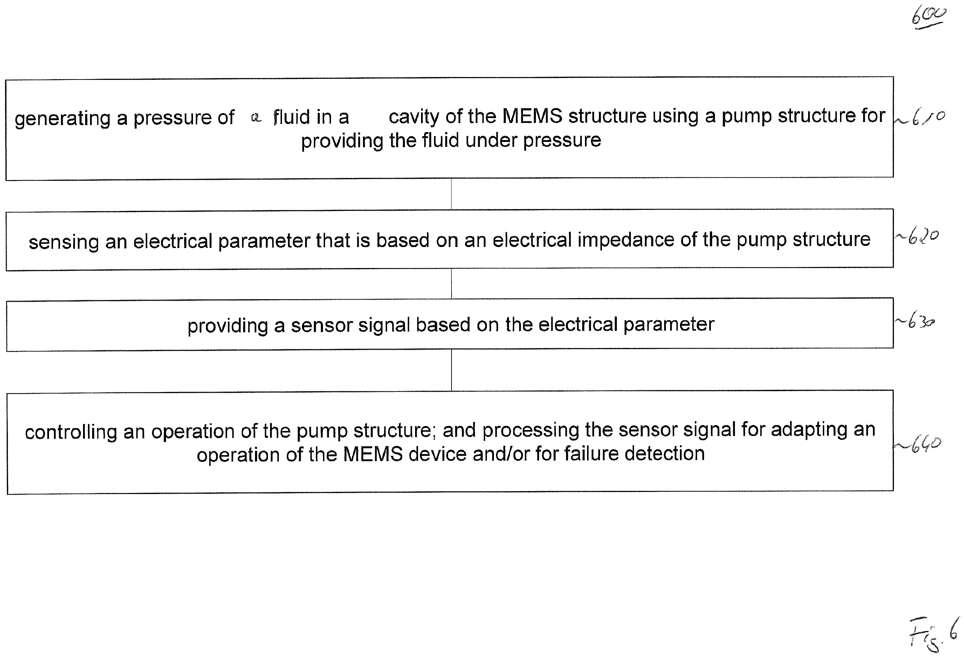

Fig. 6 shows a schematic flow chart of a process or method 600 according to an embodiment. In 610, there is generated a pressure of a fluid in a cavity of the MEMS structure using a pump structure for providing the fluid under pressure. In 620, an electrical parameter is sensed, the electrical parameter being based on an electrical impedance of the pump structure. In 630, a sensor signal is provided based on the electrical parameter. In 640, an operation of the pump structure is controlled and the sensor signal is processed for adapting an operation of the MEMS device and/or for failure detection.

In embodiments, a pumping speaker is combined with an impedance sensor and/or a pressure sensor. Alternatively or in addition, other sensors may be used in the combination.

According to a first aspect, a MEMS device comprises:
a cavity (12) for emitting a fluid (14);
a pump structure (16; 16') configured for generating a pressure of the fluid (14) in the cavity (12) and for providing the fluid (14) under pressure;
a sensor unit (22; 22') configured for sensing an electrical parameter that is based on an electrical impedance of the pump structure (16; 16'); and for providing a sensor signal (24) based on the electrical parameter;
a control unit (26) configured for controlling an operation of the pump structure (16; 16'); wherein the control unit (26) is configured for processing the sensor signal (24) for adapting an operation of the MEMS device and/or for failure detection.

According to a second aspect referring to the first aspect the MEMS device comprises a MEMS speaker; wherein the pump structure (16; 16') is configured for providing the fluid (14) with a sound pressure level.

According to a third aspect referring to the first or second aspect the pump structure (16') comprises at least one valve structure (32), e.g., a shutter and perforated structure and a volume displacing structure (34) such as a non-perforated or perforated membrane configured for generating the pressure in the cavity (12) and for releasing the fluid (14) by switching between a first state of the valve structure (32) in which the valve structure (32) provides a first fluidic resistance for the fluid (14) and a second state in which the valve structure (32) provides a second fluidic resistance for the fluid (14); wherein the first fluidic resistance is higher when compared to the first fluidic resistance.

According to a fourth aspect referring to the aspect three, the sensor unit (22; 22') is configured for measuring the electrical parameter at the valve structure (32) and/or at the volume displacing structure (34).

According to a fifth aspect referring to the first aspect 3 or 4, the control unit (26) is configured for controlling at least one of:
- a timing or phase of a movement of the volume displacing structure (34) with respect to a timing of a movement of the valve structure (32);
- a timing or phase of a movement of the valve structure (32) with respect to a timing of a movement of the volume displacing structure (34);
- an operation frequency of the pump structure (16; 16'); and
- an operational voltage of the MEMS device.

According to a sixth aspect referring to one of the previous aspects, the control unit (26) is configured for measuring the electrical parameter as a single measurement value or by measuring a plurality of instances of the electrical parameter and by relating the plurality of instances.

According to a seventh aspect referring to aspect 6, the control unit (26) is configured for measuring the plurality of instances of the electrical parameter and for relating the plurality of instances to obtain a signature of the measurement values;
wherein the control unit (26) is configured for evaluating the signature to determine a target operation, e.g., a deviation from a specific state and/or the specific state itself, and for adjusting the operation based on the signature.

According to an eighth aspect referring to one of the previous aspects, the control unit (26) is configured for evaluating a number of pulses e.g. charge transfers provided by an electrical circuit to drive the pump structure itself (16; 16') or driving a circuit driving the pump structure to determine the signature and to derive, from the signature information related to settling behavior of the pump structure (16; 16').

According to a ninth aspect referring to aspect 7 or 8, the control unit (26) is configured for determining different signatures for different operating frequencies of the pump structure (16; 16') and for comparing the different signatures to obtain an evaluation result; and for adapting the operation of the MEMS device and/or for failure detection based on the evaluation result.

According to a tenth aspect referring to one of the previous aspects, the sensor unit (22; 22') is configured for sensing one of a voltage, current and a charge between electrodes of the pump structure (16; 16'), an impedance between electrodes of the pump structure (16; 16'), and a parameter, e.g., a signature, a count or the like, related to an electrical charge of the pump structure (16; 16') to provide the sensor signal (24).

According to an eleventh aspect referring to one of the previous aspects wherein the control unit (26) is configured for evaluating a voltage, charge, or current at electrodes forming an electrical capacitor structure within the pump structure (16; 16').

According to a twelfth aspect referring to one of the previous aspects, the MEMS device comprises a pressure sensor (66) configured for providing a sensor signal (24) that is based on a pressure sensed by the pressure sensor.

According to a thirteenth aspect referring to one of the previous aspects, the control unit (26) comprises an application specific integrated circuit, ASIC, wherein the sensor unit (22; 22') is a part of the ASIC.

According to a fourteenth aspect referring to one of the previous aspects, the pump structure (16; 16') is a part of a loudspeaker structure configured for providing a first sound in the fluid (14) having a first frequency range; wherein the MEMS device comprises a membrane structure (64) configured for providing a second sound in a second frequency range higher than the first frequency range and based on a deflection of the membrane structure (64); wherein the MEMS device is configured for providing a combination of the first sound and the second sound.

According to a fifteenth aspect a method (600) for operating a MEMS device comprises:
generating (610) a pressure of the fluid in a cavity of the MEMS structure using a pump structure for providing the fluid under pressure;
sensing (620) an electrical parameter that is based on an electrical impedance of the pump structure;
providing (630) a sensor signal based on the electrical parameter;
controlling (640) an operation of the pump structure; and processing the sensor signal for adapting an operation of the MEMS device and/or for failure detection.

According to a sixteenth aspect a MEMS device comprises:
a cavity for receiving and emitting a fluid;
a pump structure configured for generating a first pressure of the fluid in the cavity and for providing the fluid under pressure;
a sensor unit configured for sensing a second pressure of the MEMS device that is related to the first pressure in the cavity, e.g., directly or indirectly;
a control unit configured for controlling an operation of the pump structure; wherein the control unit is configured for processing the sensor signal for adapting an operation of the MEMS device and/or for failure detection.

According to a seventeenth aspect referring to the sixteenth aspect, the second pressure is the first pressure.

According to an referring to one of aspects 16 or 17 the second pressure relates to an in-ear pressure, a backvolume pressure, an emitted sound pressure and/or an ambient pressure.

According to an eighteenth aspect referring to one of aspects 16 or 17 the control unit is configured for determining a deviation of a fluid flow when compared to a reference value and for adapting the operation for at least partially compensating the deviation.

According to a nineteenth aspect referring to one of aspects 16 to 18 the pump structure is configured for providing the fluid into a front volume of the MEMS device; wherein the pump structure comprises a first membrane structure fluidically coupled between the front volume and a first back volume of the pump structure; wherein the sensor unit comprises a second membrane structure fluidically coupled between a second back volume and the front volume; wherein a pressure in the front volume is related to a deflection of the second membrane structure.

According to a twentieth aspect referring to aspect 19 the first back volume and the second back volume are fluidically connected to each other or comprise a common volume.

According to a twenty-first aspect referring to aspect 19 the first back volume and the second back volume are fluidically disconnected from each other.

According to a twenty-second aspect referring to one of aspects 16 to 21 the sensor unit is configured for sensing the second pressure as at least one of:
- a differential pressure between a front volume of the MEMS device, e.g., an ear-volume and a back volume of the pump structure;
- an absolute pressure in a front volume of the MEMS device, e.g., an ear volume;
- an absolute pressure in a back volume of the MEMS device;

According to a twenty-third aspect referring to aspect 16 to 22 the sensor unit is configured for sensing the second pressure in at least one of:
- a sub-acoustic frequency range such as infrasound;
- an ultrasound frequency range such as ultrasound;
- an audible bass-related frequency range, e.g., frequencies of above 15 Hz and/or below 1 kHz or below 200 Hz;

According to a twenty-fourth aspect referring to one of aspects 16 to 23 the control unit is configured for determining, from the sensor signal, information related to a static pressure build-up in the MEMS device and for adapting the operation for at least partially compensating the static pressure build-up and related distortion.

According to a twenty-fifth aspect referring to one of aspects 16 to 24 the control unit is configured for determining an ultrasound sound pressure level based on the sensor signal e.g., in the ear; wherein the control unit is configured for adjusting an operation frequency of the pump structure and/or a phase shift of the pump structure to at least partially compensate for the ultrasonic sound pressure level or the phase shift for adapting the operation of the MEMS device.

According to a twenty-sixth aspect referring to one of aspects 16 to 25 the control unit comprises an application specific integrated circuit, ASIC, wherein the sensor unit is a part of the ASIC.

According to a twenty-seventh aspect referring to one of aspects 16 to 26, wherein the pump structure is a part of a loudspeaker structure configured for providing a first sound in the fluid having a first frequency range; wherein the MEMS device comprises a membrane structure configured for providing a second sound in a second frequency range higher than the first frequency range and based on a deflection of the membrane structure; wherein the MEMS device is configured for providing a combination of the first sound and the second sound.

Embodiments allow to obtain a high sensitivity along with a low power consumption that are both crucial for portable, battery-powered, miniaturized systems, in particular, when operating them in a continuous operation, especially with a continuous operation of vents/shutters that cause constant actuator activity, losses and battery drain. Battery life can be maximized or optimized or enhanced by operating pumping speaker vents, e.g., shutters and/or membranes at or close to a resonance frequency to reduce power and losses for the constant actuation. Such resonances may be found, by determining measurement values and/or a signature. In particular, when determining a signature at different frequencies, an optimum or at least a most suitable signature amongst the determined ones may be found. That is, manufacturing tolerances that may lead to variations of a vent structure, a shutter structure and/or a membrane structure and their resonances and/or deviations from a system symmetry that may lead to directional mismatch of flow rates and flow depended pressure build-up adverse to a pump throughput and linearity may be addressed. Moreover these resonance in particular, in connection with aging effects and/or drift of other system conditions or properties as e.g. temperature, ambient pressure, and moisture can drift. Such variations and drift be compensated as described above and the system be continuously operated in at peak performance.

Embodiments allow to achieve a high pumping speaker efficiency, e.g., measured by dB_{SPL}/mW, a high-fidelity and/or a high compactness, .e.g., measured by dB_{SPL}/mm³. In this context, embodiments allow to sense and adjust a vent/shutter and/or membrane operation to resonance by determining the electrical impedance and associated results. This may allow to match flow-rates into and out of the ear to avoid rectifying effects leading to static pressure build-up.

Embodiments allows to provide a fully integrated on-chip concept that may be desirable as other sensors such as position or acceleration sensors are often required for auralization, but the integration of multiple different sensors increases at least the system assembly costs, the solution size and may introduce manufacturing variations. Embodiments described herein may allow to avoid such efforts.

Embodiments relate to a sensing of a vent or shutter resonance and movement with a shutter drive or sense electrodes to measure an impedance between a drive or (dedicated) position sense electrodes, the impedance varying with a position of the element, especially in connection with electrostatic and/or piezoelectric elements such as microphones.

A measurement of an impedance variation can be obtained during an operation, during a test and/or during a calibration sweep. That is, the adaptation may be implemented once, in intervals, responsive to a trigger and/or may compensate for deviations arising during one or more instances of time of a lifetime of a MEMS device. Embodiments allow for adjusting a drive, e.g., a timing a voltage or the like, of a drive and/or to detect failures or defects. Embodiments allow, as an alternative or in addition, to sense a pump membrane resonance and/or a displacement with the membrane drive or sensor electrodes. Similar to an impedance measurement between membrane drive or sense electrode to vents, advantages may be obtained.

An integration on impedance an optionally a pressure sensing circuit into an ASIC may enable a direct feedback from external sensors. MEMS drive signals may be adjusted based on a sound pressure and/or an impedance sensor reading. Additional sensing capabilities such as a pressure sensing can be integrated for a one-in-all solution.

Additional sensors may, according to embodiments, be integrated in a package or a MEMS. This allows to achieve a one-chip solution to enhance a functionality of a micro speaker combined with an ASIC by using additional sensor, amongst them pressure, microphone, position, acceleration, vibration, ultrasound or the like to enable a direct feedback and a dynamic closed loop control as well as other effects, e.g., auralization.

According to embodiments, it is enabled to sensor a pump membrane resonance and displacement with membrane drives or sense electrodes. For example and as described in connection with Fig. 3a, a piezoelectric actuated membrane MEMS, a part of the electrode area can be used for sensing and actuation with single cost of effective piezoelectric material layers on a MEMS device. A pressure sensor may be generated with a same or similar process as a process to provide for the speaker membrane, i.e., the volume displacing element.

In embodiments, an audible or inaudible sound pressure level, e.g., in the ear, can be controlled by calibration or closed loop control. In view of the frequencies being used to control one or more elements of the MEMS, effects in the respective frequency range may be addressed, e.g., with low or even no additional efforts.

With regard to an implementation example of a combined sensing/drive circuit for an impedance measurement in a system-on-chip based on a charge pump driver for vent actuations. For example, a periodic measurement of the counts respectively charge transfers and/or time required to achieve a certain reference or target voltage on the actuator or pump electrode respectively capacitor may be facilitated hardware and power efficiently, e.g., by using a a counter configured for counting pulses e. g. a clock or signal driving a charge pump circuit. According to an embodiment, such a counter may be reset at the beginning of each charging cycle within the pumping cycle to reset its initial value. Alternatively or in addition the difference of counter values when compared to the prior cycle may be evaluated. The achievement of a voltage, charge or position reference level of the pump respectively actuator capacitance respectively an electrode may gate either charge-pump, counter, or both and may thus yield the number of counts for single or multiple pumping cycles.

The achievement of a reference level may be determined e.g. by using a switched Cap circuit or programmable voltage divider in combination with a comparator or Analog-to-Digital Converter and may allow for information to be used by the control unit. The number of counts may be transferred and stored into a register 73 or memory unit for post-processing and/or may be directly be postprocessed.

An optional and possibly sporadic measurement of an actual battery supply voltage level may be used to consider and compensate the effect of battery drain during operation. Alternatively, or in addition, the counts or re-charging time can also be measured on a circuit supplying the driver circuit of MEMS actuator resp. electrode with a regulated voltage.

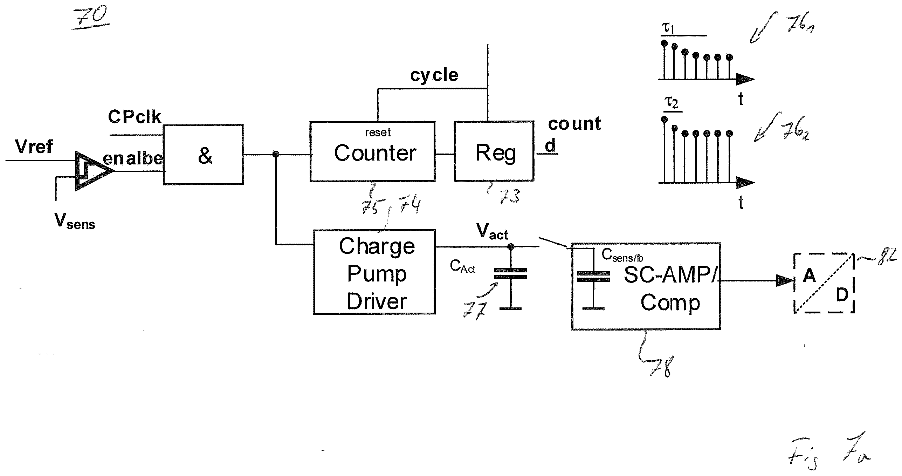

Referring to this, Fig. 7a shows a schematic block diagram of an implementation example of a combined sensing and driver circuit 70 that may be used for an impedance measurement in embodiments described herein, e.g., in a System-on-Chip, SoC, based on a charge pump driver for vent actuations. A charge pump 74 may be used

A voltage that Vₛₑₙₛ may be derived from a voltage V_{act} applied to the load/impedance, e.g., by simple attenuation. Such attenuation is not mandatory, but may help to save significant power with low-voltage devices and circuitry. This may provide for a relationship between V_{act} and Vₛₑₙₛ which may at least in parts, control how long the charge pump 74 is enabled and how many clock pulses are clocking both charge pump 75 and counter 75 and how much charge is delivered to load before a constant threshold voltage V_{ref} is achieved. This may indicate how far the load/impedance V_{act} is charged.

This can be implemented in various ways. For example, Vₛₑₙₛ may be obtained or generated from V_{act} by:
a) using a voltage divider connected to V_{act} formed by the capacitance of the load C_{act} and a switched cap circuit 78 comprising a capacitance Cₛₑₙₛ to form a combined low power capacitive/resistive voltage divider generating Vₛₑₙₛ
b) As output of an attenuating circuit as
   a. an analog output voltage Vₛₑₙₛ of an attenuating SC-Amplifier or
   b. by digitizing the Analog output voltage of SC-Amplifier with an analog-to-digital, ADC, and comparing the result to a digital reference. The shown comparator may be implemented analog or digital in nature; and/or
   c. with an attenuating SC-Comparator

Such a concept may allow to consider the battery status when evaluating counts of the charge pump, e.g., to provide for a sort of error correction. Embodiments may further consider a drop of battery voltage during operation of a MEMS in accordance with embodiments. For example, if supplied directly from a battery, a decrease in energy stored in the battery may lead to a decrease in the battery voltage. For example, at reduced battery voltage less charge is likely to be transferred when compared with a high battery voltage and thus more counts may be required to charge a target to a reference level even in face of a constant load and impedance. Such an effect and measurement principle is shown in Fig. 7a where a diagram 76, schematically illustrates a low amount of charge transferred with pulses sat low battery voltage and a diagram 76₂ illustrates a higher amount of charge transferred by use of a same number of pulses of the charge pump at high battery voltage. Such a concept may apply to measurements of charging valves and/or membrane. Alternatively, if supplied from a sub-regulation, no drop is to be expected.

When referring again to the possibility to consider the battery supply voltage level, according to embodiments, a MEMS may be adapted to adjust operation based thereon. For example, a described optional, e.g., triggered, cyclically or periodic measurement of the supply voltage may be performed using a sensing mechanism. This may include to convert a measured battery voltage vdd to a digital value, e.g., using an analog-to-digital, ADC, conversion. Alternatively or in addition, a MEMS may be adapted for a measurement of a voltage on or between an actuator such as an electrode or piezo-element or a capacitor of the pump, e.g., using the switched cap circuit 78 and/or a different programmable voltage divider. This does not exclude optional additional adjustments of, e.g., pulse durations, pause times between pulses, a duty cycle or the like, e.g., at an optional ADC 82.

In other words, with regard to an implementation example of a combined sensing/drive circuit for an impedance measurement in a system-on-chip such a circuitry may be based on a charge pump driver for vent or membrane actuations. A periodic measurement of the counts respectively charge transfers or time required to achieve a certain reference or target voltage on the actuator or pump electrode respectively capacitor can be facilitated hardware and power efficiently with a counter counting pulses e.g. a clock or logic signal driving a charge pump circuit. For example, the counter can be reset at the beginning of each charging cycle within the pumping cycle to reset its initial value (or alternatively the difference of counter values vs the prior cycle can be evaluated). The achievement of a voltage, charge or position reference level of the pump respectively actuator capacitance respectively an electrode can gate either charge-pump, counter, or both and thus yield the number of counts for single or multiple pumping cycles. The achievement of a reference level can be determined e.g. by using a switched Cap circuit or programmable voltage divider in combination with a comparator or Analog-to-Digital Converter and may allow for information to be used by the control unit. The number of counts can be transferred and stored into a register or memory unit for post-processing or be directly be postprocessed. A sporadic measurement of an actual battery supply voltage level may be used to consider and compensate the effect of battery drain during operation. Alternatively, or in addition, the counts or re-charging time can be measured on a circuit supplying the driver circuit of MEMS actuator resp. electrode with a regulated voltage.

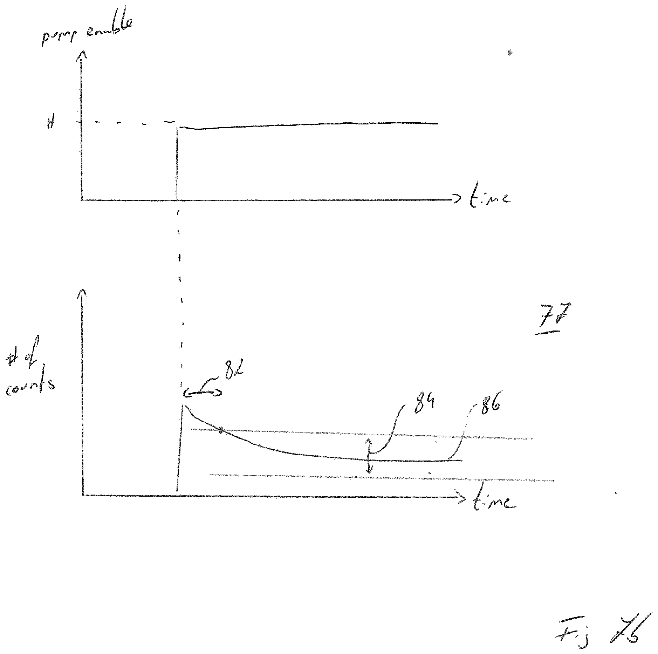

Fig. 7b shows a schematic representation of a plot 77 over a time at the ordinate to provide a link to Fig. 7a and to a sense signal such as a pump enable signal to activate the pump of the MEMS at status high, H.

Diagram 77 shows, on a same time axis, a number of counts, e.g., of the charge pump 74 to reach, e.g., a certain threshold for the pump to be driven. A settling time 82 may describe a time until a setting to a certain criteria is obtained, e.g., settling within a corridor 84 with regard to a steady state 86 of the pump and/or a slope. Such a settling time parameter measurement may be swept over pump operation frequency to extract optimum operation conditions with respect to pumping frequency and phase delay or timing.

Fig. 7b may be considered as a depiction of a resonance and frequency characteristic extraction. For one or a set of parameter combinations including the operation frequency of the pump the settling behavior during a transient change at startup may be monitored. As an alternative or in addition, an amplitude, frequency and phase behavior may be monitored.

In an implementation that may provide for an alternative, instead of an actuator, the amplifier related to the actuator may be connected to an output of a charge pump boost converter or sub-regulator. However, this concept is not limited to this specific implementation, the approach can be extended to other power converters and actuator drive implementations. Embodiments relate to an impedance sensing and a pumping speaker (or combo, e.g., with a tweeter) with a capability to sense a position, movement and/or a resonance of flow modulating or a direct element such as a vent or shutter with (drive or sense) electrodes based on an impedance measurement between electrodes and circuit to adjust the drive, e.g., a timing, voltage or the like, and/or to detect failure or defects during operation, tests or calibration sweeps or combinations thereof. Alternatively or in addition, a pumping speaker (or combo) is provided with the capability to sense a position, movement and/or resonance of volume displacing elements such as a membrane with (drive or sense) electrodes based on impedance measurements between electrodes and a circuit to adjust a drive (timing/voltage or the like) and/or detect failure or defects during operation, tests or calibration sweep.

Embodiments allow for a closed loop control. A pumping speaker system (or combo) with a regulation or adjustment of a pump operation being based on sensing pressure variations and/or static pressure built-up in size and beyond the audible range, e.g., in the infra or ultra-sonic frequency beside the audible spectrum, may be obtained. According to such embodiments, a MEMS device includes a pressure sensor. According to embodiments, a pumping speaker (or combo) actuator MEMS, e.g., comprising a membrane and/or a vent, is provided having an integrated pressure/sound sensor comprising a membrane, sensing electrodes and a cavity. A pressure may be sensed with electro-static or piezo-electric transducers or other transducers and a static pressure may be captured and/or pressure fluctuations within and/or beyond the audible range may be captured, e.g., in the ultrasonic and/or infra-sound range.

According to an embodiment, a single-chip ASIC is provided, where a speaker drive for a pump, an Advanced Digital Sound Reconstruction, ADSR, a tweeter and/or a combo thereof, may be extended with an integrated analog front end with a capability to sense a position, movement and/or resonance of a flow modulating or directing elements such as a vent and/or a shutter and/or a membrane with drive or sense electrodes based on an impedance measurement between electrodes and/or a circuit to adjust a drive, e.g., a timing, a voltage or the like, and/or to detect failures or defects during operation, tests or calibration sweeps. Alternatively or in addition, such a single-chip ASIC being extended with an integrated analog-front end may be provided for sound monitoring such as a static pressure, an infrasound, ultra-sound using appropriate sensitive microphone features. The integration may enable the implementation of a continuous closed-loop operation or a periodic calibration to compensate for, e.g., non-linearities of the speaker and/or to enable additional features, e.g., for auralization.

Embodiments may allow for performing measurements, e.g., of the pump operation during a variation of an ambient condition, e.g., a pressure, a temperature or the like, and to check an adjustment of operation frequency with regard to an expected shift in the resonance frequency.

Although some embodiments have been described with regard to a true wireless system, TWS speaker, also non-pumping TWS speakers, e.g., with limited bass performance or larger size, may be used in connection with the present disclosure. Embodiments allow for using a pumping speaker without calibration or control loop and consequently to omit a pressure control and highly varying audio performance and power dissipation. However, a calibration is not precluded.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Depending on certain implementation requirements, embodiments of the disclosure can be implemented in hardware or in software. The implementation can be performed using a digital storage medium, for example a floppy disk, a DVD, a CD, a ROM, a PROM, an EPROM, an EEPROM or a FLASH memory, having electronically readable control signals stored thereon, which cooperate (or are capable of cooperating) with a programmable computer or controller system such that the respective method is performed.

Some embodiments according to the disclosure comprise a data carrier having electronically readable control signals, which are capable of cooperating with a programmable computer system, such that one of the methods described herein is performed.

Generally, embodiments of the present disclosure can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

In other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein.

A further embodiment of the inventive method is, therefore, a data stream or a sequence of signals representing the computer program for performing one of the methods described herein. The data stream or the sequence of signals may for example be configured to be transferred via a data communication connection, for example via Bluetooth transmission or the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The above described embodiments are merely illustrative for the principles of the present disclosure. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

## Claims

1. A MEMS device comprising:
a cavity (12) for emitting a fluid (14);
a pump structure (16; 16') configured for generating a pressure of the fluid (14) in the cavity (12) and for providing the fluid (14) under pressure;
a sensor unit (22; 22') configured for sensing an electrical parameter that is based on an electrical impedance of the pump structure (16; 16'); and for providing a sensor signal (24) based on the electrical parameter;
a control unit (26) configured for controlling an operation of the pump structure (16; 16'); wherein the control unit (26) is configured for processing the sensor signal (24) for adapting an operation of the MEMS device and/or for failure detection.

2. The MEMS device of claim 1, wherein the MEMS device comprises a MEMS speaker; wherein the pump structure (16; 16') is configured for providing the fluid (14) with a sound pressure level.

3. The MEMS device of claim 1 or 2, wherein the pump structure (16') comprises a valve structure (32) and a volume displacing structure (34) configured for generating the pressure in the cavity (12) and for releasing the fluid (14) by switching between a first state of the valve structure (32) in which the valve structure (32) provides a first fluidic resistance for the fluid (14) and a second state in which the valve structure (32) provides a second fluidic resistance for the fluid (14); wherein the first fluidic resistance is higher when compared to the first fluidic resistance.

4. The MEMS device of claim 3, wherein the sensor unit (22; 22') is configured for measuring the electrical parameter at the valve structure (32) and/or at the volume displacing structure (34).

5. The MEMS device of claim 3 or 4, wherein the control unit (26) is configured for controlling at least one of:
• a timing or phase of a movement of the volume displacing structure (34) with respect to a timing of a movement of the valve structure (32);
• a timing or phase of a movement of the valve structure (32) with respect to a timing of a movement of the volume displacing structure (34);
• an operation frequency of the pump structure (16; 16'); and
• an operational voltage of the MEMS device.

6. The MEMS device of one of previous claims, wherein the control unit (26) is configured for measuring the electrical parameter as a single measurement value or by measuring a plurality of instances of the electrical parameter and by relating the plurality of instances.

7. The MEMS device of claim 6, wherein the control unit (26) is configured for measuring the plurality of instances of the electrical parameter and for relating the plurality of instances to obtain a signature of the measurement values;
wherein the control unit (26) is configured for evaluating the signature to determine a target operation and for adjusting the operation based on the signature.

8. The MEMS device of claim 7, wherein the control unit (26) is configured for evaluating a number of pulses e.g. charge transfers provided by an electrical circuit to drive the pump structure itself (16; 16') or driving a circuit driving the pump structure to determine the signature and to derive, from the signature information related to settling behavior of the pump structure (16; 16').

9. The MEMS structure of claim 7 or claim 8, wherein the control unit (26) is configured for determining different signatures for different operating frequencies of the pump structure (16; 16') and for comparing the different signatures to obtain an evaluation result; and for adapting the operation of the MEMS device and/or for failure detection based on the evaluation result.

10. The MEMS device of one of previous claims, wherein the sensor unit (22; 22') is configured for sensing one of a voltage, a current and a charge between electrodes of the pump structure (16; 16'), an impedance between electrodes of the pump structure (16; 16'), and a parameter related to an electrical charge of the pump structure (16; 16') to provide the sensor signal (24).

11. The MEMS device of one of previous claims, wherein the control unit (26) is configured for evaluating a voltage, a charge and/or a current at electrodes forming an electrical capacitor structure within the pump structure (16; 16')

12. The MEMS device of one of previous claims, comprising a pressure sensor (66) configured for providing a sensor signal (24) that is based on a pressure sensed by the pressure sensor.

13. The MEMS device of one of previous claims, wherein the control unit (26) comprises an application specific integrated circuit, ASIC, wherein the sensor unit (22; 22') is a part of the ASIC.

14. The MEMS device of one of one of previous claims, wherein the pump structure (16; 16') is a part of a loudspeaker structure configured for providing a first sound in the fluid (14) having a first frequency range; wherein the MEMS device comprises a membrane structure (64) configured for providing a second sound in a second frequency range higher than the first frequency range and based on a deflection of the membrane structure (64); wherein the MEMS device is configured for providing a combination of the first sound and the second sound.

15. Method (600) for operating a MEMS device, the method comprising:
generating (610) a pressure of the fluid in a the cavity of the MEMS structure using a pump structure for providing the fluid under pressure;
sensing (620) an electrical parameter that is based on an electrical impedance of the pump structure;
providing (630) a sensor signal based on the electrical parameter;
controlling (640) an operation of the pump structure; and processing the sensor signal for adapting an operation of the MEMS device and/or for failure detection.
